Europäisches Patentamt

European Patent Office

Office européen des brevets

(11) Publication number: **0 150 898**
A2

# EUROPEAN PATENT APPLICATION

(21) Application number: **85300040.4**

(22) Date of filing: **03.01.85**

(51) Int. Cl.⁴: **B 01 J 21/16,** B 01 J 29/02,
C 07 C 1/04, C 07 C 29/15

(30) Priority: **06.01.84 GB 8400271**

(43) Date of publication of application: **07.08.85**
**Bulletin 85/32**

(84) Designated Contracting States: **BE DE FR GB IT NL**

(71) Applicant: **The British Petroleum Company p.l.c.,
Britannic House Moor Lane, London EC2Y 9BU (GB)**

(72) Inventor: **Atkins, Martin Philip, The British Petroleum Co.
p.l.c. Chertsey Road, Sunbury-on-Thames Middlesex,
TW16 7LN (GB)**
Inventor: **Ashton, Antony Gary, The British Petroleum Co.
p.l.c. Chertsey Road, Sunbury-on-Thames Middlesex,
TW16 7LN (GB)**

(74) Representative: **Harry, John et al, BP INTERNATIONAL
LIMITED Patents Division Chertsey Road,
Sunbury-on-Thames Middlesex TW16 7LN (GB)**

(54) **Novel pillared interlayered clays, processes for their production and uses thereof.**

(57) The invention relates to silanised pillared interlayered
clays which are produced by hydrolysing a hydrolysable silicon
compound in the liquid or vapour phase in the presence of a pil-
lard interlayered clay or a precursor thereof. The resulting si-
lanised clays are more active as catalysts and/or more stable
than the clays from which they are derived.

1

## NOVEL PILLARED INTERLAYERED CLAYS, PROCESSES FOR THEIR PRODUCTION AND USES THEREOF

The present invention relates to novel pillared interlayered clays, processes for their production and to uses thereof.

Natural and synthetic clays having a lamellar structure with interlamellar spaces disposed between the lamellar layers are well known. Smectites, such as bentonite and montmorillonite are a class of clays possessing such a lamellar structure. Montmorillonite has an idealised stoichiometric composition corresponding to $Na_{0.67}[Al_{3.33}Mg_{0.67}](Si_8)O_{20}(OH)_4$. Structurally it comprises a central layer containing octahedrally coordinated aluminium and magnesium in the form of their oxides and hydroxides sandwiched between two layers containing tetrahedrally coordinated silicon essentially in the form of its oxide. Normally in nature cations are present to compensate for the charge imbalance caused by isomorphous substitution of $Mg^{2+}$ for $Al^{3+}$ in the octahedral layer, and/or $Al^{3+}$ or other ions for $Si^{4+}$ in the tetrahedral layers. The octahedral and tetrahedral regions are tightly bound together to form a lamellar layer. The space between these lamellar layers, i.e. the interlamellar space, in natural clays is normally occupied by exchangeable $Ca^{2+}$ or $Na^+$ ions. The distance between the interlamellar layers can be increased substantially by adsorption of various polar molecules such as water, ethylene glycol, amines etc, which enter the interlamellar space and in doing so push the layers apart. It is generally recognised that such clays when heated in air suffer irreversible collapse of their layered structures at temperatures of 180°C and above, the actual value of this temperature depending upon the nature of the exchangeable cation present in the interlamellar region of the structure. The collapse of the layered structure is associated with a loss in the catalytic activity of the clay. Layered clays can also suffer from the disadvantage that they suffer particle/granule instability in the presence of polar liquids such as methanol or ethanol. Generally, this does not impair catalytic activity in batch or continuous reactions in which the catalyst particles are subject to agitation, thereby maintaining them in suspension. However, the catalyst particles have at some stage to be separated from the reactor product by filtration, centrifugation or other solid/liquid separation methods. For fixed bed operation, the layered clay catalyst is preferably mixed with suitable binders to ensure granule or pellet stability.

Also known from, for example, U.S. Patents Nos. 4238364; 4176090; 4271043 and 4248739 are stabilised pillared interlayered clays in which the layers are separated and supported by "pillars" of monomeric, oligomeric or polymeric species.

USP 4238364 describes and claims a novel cracking catalyst comprising a crosslinked smectite framework material functionalised with acidic ions selected from the group consisting of the ions of hydrogen and rare earth elements and a method for preparing the material which comprises:

preparing an acidic form of smectite including ions selected from the group consisting of hydrogen, cerium, gadolinium, lanthanum, neodymium, praseodymium, and samarium; crosslinking the acidic form of smectite with oligomeric species of aluminium hydroxide and

stabilising the crosslinked acidic form of smectite.

USP 4,271,043 describes and claims a process for preparing a pillared interlayered clay product having a high degree of ion exchange capacity which comprises:

(a)  reacting a smectite clay with a mixture of a polymeric cationic hydroxy metal complex selected from the group consisting of polymeric cationic hydroxy aluminium and zirconium complexes and water to obtain a pillared interlayered smectite;

(b)  calcining said interlayered smectite to obtain an interlayered clay product having greater than 50% of its surface area in pores less than 30 Angstroms in diameter: and

(c)  reacting said calcined interlayered clay product with a base to increase the ion exchange capacity thereof.

USP 4,248,739 describes and claims a method for preparing a pillared interlayered smectite clay product wherein a smectite clay is reacted with a mixture of polymeric cationic hydroxy metal complex and water to obtain a pillared interlayered smectite having greater than 50 percent of its surface area in pores of less than 30 Angstroms in diameter after dehydration wherein the improvement comprises reacting said smectite with a high molecular weight cationic hydroxy metal complex and copolymers thereof having a molecular weight of from about 2,000 to 20,000.

Both USP 4,271,043 and USP 4,248,739 describe the use of high molecular weight polymers which may be prepared by copolymerising an aluminium or zirconium metal complex with a copolymerising reactant which may be included in the reaction mixture as sodium silicate, zirconium chloride, boric acid or sodium borate. There is no disclosure of the use of sodium silicate other than as a component of a high molecular weight polymer, as used in "pillaring" the layered clay. The pillared interlayered clays so produced are said to be useful as sorbents, catalysts and catalyst supports. It is claimed that the pillaring treatment, irrespective of the nature of the pillars, improves the hydrothermal stability of the catalyst to regeneration cycles in cracking reactions.

We have now found that the chemical and/or hydrothermal and/or thermal stability of pillared interlayered clays can be improved and, furthermore, their activity and/or selectivity as catalysts in certain reactions can be considerably improved.

Accordingly, the present invention provides a silanised pillared interlayered clay comprising a pillared interlayered clay chemically modified by incorporation therein of a silicon-containing residue.

According to another aspect of the present invention there is provided a process for the production of a silanised pillared interlayered clay which process comprises hydrolysing in the presence of a pillared interlayered clay or a precursor thereof a hydrolysable silicon compound.

In order to avoid any confusion as to the stage at which a material is a pillared interlayered clay and not merely a precursor thereof, a material incorporating a solvated pillaring moiety will be regarded throughout this specification as a precursor of a pillared interlayered clay and a material which has been calcined, will be regarded as a pillared interlayered clay. In the particular context of reacting an interlayered clay with a solution of a polymeric cationic hydroxy metal complex, the product of this reaction containing solvated polymeric cationic hydroxy metal complex is regarded as a precursor of a pillared interlayered clay, which after calcination becomes a pillared interlayered clay.

Suitable pillared interlayered clays and methods for preparing them or precursors thereof (Step A) are described in for example the aforesaid US Patents Nos. 4,176,090, 4,238,364, 4,248,739 and 4,271,043. Alternatively, the pillared interlayered clays, for example the boria pillared interlayered clays or precursors thereof prepared as described in our copending European application publication No. 0130055 (BP Case No. 5612) or the beryllium pillared interlayered clays or precursors thereof prepared as described in our copending UK application No. 83 33614 (BP Case No. 5740) may be used. Of the aforesaid pillared interlayered clays, the alumina pillared interlayered clays as described in US Patents Nos. 4,176,090, 4,238,364, 4,248,739 and 4,271,043 are preferred.

In a preferred embodiment, the silanised pillared interlayered clay may be produced by the steps comprising:

(A)  forming the precursor of a pillared interlayered clay,

(B) recovering the precursor of the pillared interlayered clay and optionally washing the recovered precursor,

(C) calcining the precursor recovered in step (B) to form the pillared interlayered clay, and

(D) in the presence of the pillared interlayered clay formed in step (C) hydrolysing a hydrolysable silicon compound.

Preferably, the precursor recovered in step (B) is washed, suitably with water, aqueous alcohol or the like. It is also preferred to dry the washed precursor, suitably at a temperature of up to about 100°C.

As regards step (C) the precursor may suitably be calcined at a temperature in the range from 200 to about 550°C, preferably about 350°C to about 450°C, suitably in air, oxygen, hydrogen or an inert gas, for example nitrogen.

It is possible to omit step (C) and produce a silanised pillared interlayered clay having higher catalytic acitivity in certain reactions than the non-silanised pillared interlayered clay, though the ommision of step (C) generally leads to clays less hydro-thermally and/or thermally stable than those resulting from the inclusion of step (C).

As regards step (D), hydrolysis of the hydrolysable silicon compound may suitably be achieved by contacting the compound in the liquid phase with a hydrolysing agent in the presence of the pillared interlayered clay under conditions which do not impair the layered structure of the clay, preferably at a temperature above ambient but below that which results in structural impairment. Suitably the temperature may be in the range from 50 to 170°C, although the upper temperature limit will depend upon the choice of hydrolysing agent and hydrolysable silicon compound.

Any suitable hydrolysable silicon compound may be employed in the process of the invention. Suitable hydrolysable silicon compounds include silicon halides and, more conveniently, the tetraalkoxysilanes, wherein the alkoxy groups may be the same or different. An example of a tetraalkoxysilane which may be used in the method of the invention is tetraethoxysilane. The amount of the hydrolysable silicon compound to be employed will depend upon the amount of silicon it is desired to incorporate into the layered clay. The hydrolysing agent may suitably be water, aqeuous alcohol, dilute aqeuous alkali or dilute aqeuous acid, and is preferably water. The hydrolysis mixture should be agitated during the hydrolysis in order to maintain a uniform dispersion. Hydrolysis may be effected over a period of from a few minutes to several days, preferably from about 30 minutes to about 6 hours.

The silanised pillared interlayered clay may thereafter be separated from the hydrolysate by conventional means, such as by centrifugation or filtration. It may thereafter be dried.

Alternatively, the pillared interlayered clay may be contacted with the hydrolysable silicon compound in the vapour phase under conditions which do not impair the layered structure of the pillared interlayered clay. Vapour phase contact may be effected either before or after the calcination step (C).

It is preferred in a final step (E) to heat the silanised pillared interlayered clay to a temperature suitably in the range from 250 to 550°C, preferably at about 400°C for a period of, for example from half to 12 hours. The heating may suitably be carried out in air, oxygen, hydrogen or an inert gas, such as nitrogen.

The silanised pillared interlayered clay may be used as a catalyst or a catalyst support in a wide variety or chemical reactions. For such use it will usually be beneficial either to exchange the cations normally associated with the clay with other cations, for example hydrogen ions and/or metal cations, and/or to impregnate the clay with acids and/or metal compounds. Ion-exchange of the clay, may suitably be effected either before or after silanation using techniques known in the art. Impregnation of the clay may suitably be effected after silanation, again using techniques known in the art.

Reactions in which silanised pillared interlayered clays may be used as catalysts include the conversion of synthesis gas to hydrocarbons and/or alcohols and reactions which are catalysed by protons, such as for example esterification, etherification, hydration and alkylation.

Operating conditions for the conversion of synthesis gas to hydrocarbons and/or alcohols are well known in the art and require no further elaboration. The use of pillared interlayered clays as catalysts in certain reactions which are catalysed by protons are described for example in our copending European applications publication Nos. 83970 and 110628 to which the reader is referred for further details of reactants and reaction conditions which may be employed in association with the silanised pillared interlayered clays of the present invention.

The silanised pillared interlayered clays may also be used as hydrocarbon conversion catalysts or as supports therefor. Hydrocarbon conversion reactions in which the silanised pillared interlayered clays may be employed as catalyst or catalyst support include catalytic cracking, hydrocracking, isomerisation and reforming.

The method of the invention will now be further illustrated by reference to the following Examples. In the Examples and the Comparison Test a stabilised pillared interlayered clay was used. This was prepared as follows:

Example A. PREPARATION OF PILLARED INTERLAYERED CLAYS

Wyoming montmorillonite (1143g) was added to a stirred suspension of CHLORHYDROL(RTM)(1143g) solution in water (20 litres). The pH of the solution was adjusted to pH 5.5 with aqueous ammonia and maintained at this pH while the mixture was heated for 1 hour at 65°C. The pillared interlayered clay obtained was separated by decantation. It was washed with water, dried at 80-100°C and calcined at 400-500°C in air.

The silicon:aluminium ratio of the product was 1.3:1 by weight and the basal $d_{001}$ spacing was 17.8 Angstroms.

PREPARATION OF SILANISED PILLARED INTERLAYERED CLAY

Example 1

The pillared interlayered clay (16g) obtained in Example A above was added to a glass flask containing tetraethoxysilane (20g). Water (40 ml) was added rapidly with vigorous stirring and the contents were maintained at about 70°C for 1 hour. The product was separated by decantation, dried in an oven at 100°C and calcined at 400°C for 4 hours.

The silicon:aluminium ratio of the product was 2.8:1 by weight and the basal $d_{001}$ spacing was 17.7 Angstroms.

Examples 2 to 4

The procedure of Example 1 was repeated except that the quantities of reactants were varied to produce silanised pillared interlayered clays in which the silicon:aluminium ratio was 10:1 (Example 2), 5:1 (Example 3) and 7:1 (Example 4) respectively.

Example 5

A portion (30g) of a pillared interlayered clay obtained by the method of Example A was placed in a tubular glass reactor centrally supported within an electrically heated furnace. The sample was purged with a flow of nitrogen (100 cm$^3$min$^{-1}$) at 300°C for 4 hours. The temperature was reduced to 150°C and Si(OMe)$_4$ (44g) was pumped into the reactor at a rate of 11gh$^{-1}$. The Si(OMe)$_4$ was vapourised and swept through the reactor using nitrogen as carrier gas (100 cm$^3$min$^{-1}$). After silanisation the product was calcined for 4h at 400°C in a stream of nitrogen.

Comparison Test 1

AlCl$_3$.6H$_2$O(403g) was dissolved in distilled water (1,000g) at 85°C (Solution A). Anhydrous Na$_2$CO$_3$ (220g) was dissolved in distilled water (1600g) at 85°C (Solution B). Solution A was rapidly stirred and Solution B quickly added over a 20 minute period. After completion of the Solution B addition, Na$_2$SiO$_3$ solution (containing 12% Na$_2$O and 30% SiO$_2$) (57g) diluted to 1,000 cm$^3$ was added to the mixture of Solutions A and B. To the resulting mixture, Wyoming montmorillonite (300g) was added and the suspension stirred for 1½h at 85°C. The solid product obtained was separated by centrifuging, washed with water, dried at 80-100°C and calcined in air at 400°C.

Comparison Test 2

The procedure of Comparision Test 1 was repeated except that the Na$_2$SiO$_3$ was replaced by Si(OEt)$_4$ (59g).

Comparison Test 3

The procedure of Comparison Test 1 was repeated except that no Na$_2$SiO$_3$ solution was added.

Comparison Tests 1 to 3 are not examples according to the present invention because the silanising (hydrolysis) and pillaring steps were performed simultaneously in Comparison Tests 1 and 2 and no silanising step was involved in Comparison Test 3.

Example 6

Wyoming montmorillonite (300g) was ion-exchanged with a solution containing AlCl$_3$.6H$_2$O(403g) and Na$_2$CO$_3$ (220g) prepared as described in Comparison Test 1 for solutions A and B respectively. The clay product, containing polyoxoaluminium cations was separated by centrifuging, washed with water and dried at 80-100°C.

The dry clay (80g) was dispersed in ethanol (200 cm$^3$). Si(OEt)$_4$(60g) was added and the mixture stirred for 1 hour at 60°C. Water (120g) was then added and the mixture stirred for a further 2 hours. The clay product was separated by centrifuging, washed with water, dried at 80-100°C and calcined at 400°C in air.

It will be noted that unlike Example 1, there was no calcination step between the exchange and the hydrolysis steps.

PROPERTIES OF SILANISED PILLARED INTERLAYERED CLAYS

(i) Physical properties

The BET surface area, the micropore volume and the mean pore diameter of the silanised pillared interlayered clays of Examples A, 1, 3 and 4 as determined by standard methods are given in Table 1.

The basal spacing (d$_{001}$), micropore volume, mean pore diameter and surface area of the silanised pillared interlayered clay of Example 6 and of the pillared interlayered clay of Example A as determined by standard methods are given in Table 2.

Comparing Table 2 with Table 1, it can be seen that omitting the calcination step after polyoxoaluminium cation exchange and before hydrolysis results in a silanised pillared interlayered clay having reduced pore volume and surface area.

(ii) Hydrothermal stability

(a) The hydrothermal stability of the silanised pillared interlayered clay obtained in Example 1 was investigated by steaming for 4 hours at 570°C and 670°C using a water vapour concentration of 25 vol %. The experiment was repeated using the pillared interlayered clay of Example A. The stability was monitored by determinations of the basal $d_{001}$ spacing, the surface area, the micropore volume and the mean pore diameter. The results are given in Table 3.

The results in Table 3 demonstrate that silanising a pillared interlayered clay results in enhanced hydrothermal stability.

(b) The hydrothermal stability of the interlayered clay products of Comparison Tests 1 to 3 was investigated by steaming at 670°C for 4 hours in a nitrogen stream containing 25 vol % of water vapour. Basal spacings and surface areas of the materials are shown in Table 4.

The results shown in Table 4 demonstrate that "in situ" treatment of the polyoxoaluminium cation exchanged material with either $SiO_3^{2-}$ or $Si(OEt)_4$ leads to reduced hydrothermal stability when compared with an alumina pillared interlayered clay, i.e. an interlayered clay exchanged with polyoxaluminium cations in the absence of $SiO_3^{2-}$ or $Si(OEt)_4$.

(iii) Stability to aqueous potassium hydroxide

The clay (20g) was placed in a chromatography column coupled to a fraction collector. KOH solution (1M) was passed through the clay bed at a flow rate of ca 20 ml $h^{-1}$. The eluent liquid was analysed for aluminium extracted during 1 h period. The clays examined in this manner were (i) the alumina pillared interlayered clay of Example A and (ii) the silanised alumina pillared interlayered clay of Example 1. The results are given in Table 5.

The results in Table 5 demonstrate that the silanised pillared interlayered clay according to the present invention is considerably more resistant to attack by alkali than the alumina pillared interlayered clay from which the silanised clay is derived.

(iv) <u>Acidity measurements by aqueous NaOH titration</u>

The clay (1g) was placed in a sealed conical flask in carbonate free deionised water (10ml) under a nitrogen atmosphere. An automatic titration system was used which transferred 0.1 cm$^3$ of 0.1M NaOH to the stirred flask, paused for 1 minute to achieve equilibrium, measured pH recorded and then added another aliquot of NaOH, the whole operation being repeated up to and beyond the end-point. The test was carried out on the alumina pillared interlayered clay of Example A and the silanised pillared interlayered clay (Si:Al=2.8:1) of Example 1.

The titre of NaOH required to reach pH 7.0 is shown in Table 6.

The results shown in Table 6 suggest that the silanised alumina pillared interlayered clay contains more acid sites than the alumina pillared interlayered clay from which it is derived.

CATALYTIC APPLICATIONS

(i) <u>Alkylation of aromatics</u>

<u>Examples 7 to 10</u>

Benzene (120g) was charged to a 250ml autoclave loaded with catalyst (5g). The autoclave was sealed and pressurised with ethylene via gas lines to 40 bars. The contents of the autoclave were stirred and heated at 200°C for 2½h. The products were than analysed by gas chromatography. The catalysts employed were the silanised alumina pillared interlayered clays of Examples A (Ex. 7) 1 (Ex. 8), 3 (Ex. 9) and 4 (Ex. 10).

The results are given in Table 7.

The results reported in Table 7 demonstrate that the activity of the silanised alumina pillared interlayered clays as catalysts in the production of ethyl benzene passes through a maximum at a silicon to aluminium ratio between 1.5:1 and 5.0:1.

<u>Examples 11 to 14</u>

The procedure of Examples 7 to 10 was repeated using as catalyst the silanised alumina pillared interlayered clay of Example 1 (Si:Al=2.8:1) which had been calcined at different temperatures as follows:

Ex. 11 – 300°C

Ex. 12 – 360°C

Ex. 13 – 400°C

Ex. 14 – 430°C

The results are reported in Table 8.

It can be seen from the results reported in Table 8 that for use as a catalyst in ethyl benzene production there is an optimum calcination temperature which lies between 360°C and about 430°C.

Example 15

The procedure of Examples 7 to 10 was repeated using as catalyst the silanised alumina pillared interlayered clay of Example 5.

The yield of ethyl benzene was 8 wt %.

Comparison Test 4

The procedure of Examples 7 to 10 was repeated using as catalyst the alumina pillared interlayered clay of Example A, from which the silanised alumina pillared interlayered clay of Example 5 was derived. The yield of ethyl benzene was 1 wt %.

This is not an example according to the invention because a silanised clay was not employed. It is included for the purpose of demonstrating that silanising a pillared clay can lead to improved catalytic activity.

Example 16

The procedure of Examples 7 to 10 was repeated using as catalyst the silanised alumina pillared interlayered clay of Example 6. The yield of ethyl benzene was 6 wt %.

This result when compared with the result of Comparison Test 4 demonstrates that silanising the precursor of an alumina pillared interlayered clay improves the catalytic activity of the ultimate clay product.

(ii) Petroleum cracking

Example 17

The silanised alumina pillared interlayered clay catalyst of Example 1 was evaluated as a petroleum cracking catalyst using the procedure described by Ciapetta and Henderson, Oil and Gas Journal, October 16, 1967, page 88. The results are shown in Table 9.

Comparison Test 5

Example 17 was repeated using as catalyst the alumina pillared interlayered clay of Example A instead of the silanised alumina pillared interlayered clay of Example 1. The results are shown in Table 9.

This is not an example according to the invention because a silanised clay was not used as catalyst. It is included for the purpose of demonstrating that silanising an alumina pillared layered clay, though reducing its activity, improves its selectivity to gasoline and results in considerably lower coke formation.

(iii)Glycol ether production

Examples 18 to 20

A three-necked round bottom flask was charged with ethanol (100g; 2.17 mol), a magnetic follower and the clay catalyst (2g). The flask was equipped with thermometer and dry ice/acetone condenser. The contents of the flask were stirred and heated at 70°C. Propylene oxide (13.2g) was added dropwise to the suspension (causing an exothermic reaction) over a period of 1 hour. Thereafter liquid products were removed from the flask and analysed by gas chromatography. Essentially quantitative epoxide conversions were obtained in all cases. The major reaction follows the equation:-

Propylene oxide + Ethanol $\rightarrow$ $CH_3CH(OR)CH_2OH$ + $CH_3CH(OH)CH_2OR$

(Primary alcohol) (Secondary alcohol)

The following clays were used:-

Ex. 18 - the silanised alumina pillared interlayered clay of Example 1 (Al:Si = 2.8:1).

Ex. 19 - a $Mn^{2+}$ exchanged silanised alumina pillared interlayered clay prepared by ion-exchange of the product of Example 1.

Ex. 20 - a $H^+$ exchanged silanised alumina pillared interlayered clay prepared by ion-exchange of the product of Example 1.

The results in terms of the wt. ratio of primary to secondary glycol ethers in the product stream are given in Table 10.

Comparison Test 6

The procedure of Examples 18 to 20 was repeated using the alumina pillared interlayered clay of Example A instead of the silanised clays. The result is given in Table 10.

This is not an example according to the invention because a silanised clay was not employed as catalyst. It is included only for the purpose of comparison.

The results reported in Table 10 demonstrate that silanising an alumina pillared interlayered clay can improve its selectivity to the primary alcohol, particularly when the silanised clay is cation-exchanged with hydrogen ions.

(iv) Conversion of synthesis gas to hydrocarbons

Example 21

The product of Example 1 was impregnated by slurrying it with an aqueous solution of ruthenium, iron and potassium ions sufficient to provide a catalyst having the composition by weight $(Ru_{0.24} Fe_{0.3}K_{0.13})$ $(Clay)_{6.8}$, followed by rotary evaporation of the water at 70°C. The catalyst was then dried in an oven at 125°C.

Synthesis gas ($CO:H_2=1:1$) was passed over the catalyst at 306°C and 20 bar with a contact time of 1.74 minutes. The conversion of carbon monoxide was 30% w/w and the selectivity to the major products was:

|  | % molar |
|---|---|
| methane | 18 |
| $C_2$ to $C_4$ hydrocarbons | 41 |
| higher hydrocarbons | 31 |
| methanol | 0.5 |
| ethanol | 1.8 |
| propanol | 0.5 |
| carbon dioxide | 6 |

Comparison Test 7

The stabilised pillared layered clay obtained in A above was impregnated by slurrying with an aqeuous solution of ruthenium, iron and potassium ions sufficient to give a catalyst of the composition by weight $(Ru_{0.24}Fe_{0.3}K_{0.13})$ $(Clay)_{6.8}$, followed by rotary

evaporation of the water at 70°C.  The catalyst was then dried in an oven at 125°C.

Synthesis gas ($CO:H_2=1:1$) was passed over the catalyst at 340°C and 20 bar with a contact time of 1.67 minutes.  The conversion of carbon monoxide was 21% w/w and the selectivity to the principal products was:-

|  | % molar |
|---|---|
| methane | 24 |
| $C_2$ to $C_4$ hydrocarbons | 56 |
| higher hydrocarbons | 7.5 |
| methanol | 0.7 |
| ethanol | 1.6 |
| propanol | trace |
| carbon dioxide | 10 |

This is not an example according to the invention because a silanised pillared interlayered clay was not used as the catalyst. It is included only for the purpose of comparison.

Comparing the results of this Test with those of Example 21, there is a higher total selectivity to hydrocarbons, a much higher selectivity to more desirable higher hydrocarbons and a lower selectivity to undesirable carbon dioxide using the catalyst incorporating the silanised alumina pillared interlayered clay of the invention.

TABLE 1

| Example | A | 1 | 3 | 4 |
|---|---|---|---|---|
| Si:Al ratio | 1.3:1 | 2.8:1 | 5.0:1 | 7.0:1 |
| BET SURFACE AREA ($m^2g^{-1}$) | 262 | 285 | 371 | 494 |
| MICROPORE VOLUME ($mlg^{-1}$) | 0.10 | 0.15 | 0.19 | 0.23 |
| MEAN PORE DIAMETER | 22.5 | 21.5 | 21.0 | 14.8 |

TABLE 2

| Example | $D_{001}$ (Angstroms) | micropore volume ($cm^3g^{-1}$) | surface area ($m^2g^{-1}$) | mean pore diameter (Angstroms) |
|---|---|---|---|---|
| 6 | 18.8 | 0.06 | 116 | 18.6 |
| A | 17.8 | 0.10 | 262 | 22.5 |

TABLE 3

| Property | Example A | | Example 1 | |
|---|---|---|---|---|
| | Steamed at 570°C | Steamed at 670°C | Steamed at 570°C | Steamed at 670°C |
| basal $d_{001}$ (Angstrom) | 17.1 | 16.8 | 17.8 | 17.4 |
| surface area ($m^2g^{-1}$) | 185 | 92 | 219 | 186 |
| micropore vol ($cm^3g^{-1}$) | 0.13 | 0.04 | 0.13 | 0.09 |
| mean pore diam (Angstroms) | 25.2 | 31.9 | 19.6 | 20.2 |

TABLE 4

| Comp. Test | Nature of Clay | $d_{001}$ (Angstroms) | Surface Area ($m^2g^{-1}$) |
|---|---|---|---|
| 1 | $Na_2SiO_3$/alumina pillared | 16.9 | 157 |
| 2 | $Si(OEt)_4$/alumina pillared | 10.1 | 90 |
| 3 | Alumina pillared | 17.0 | 197 |

0150898

17

TABLE 5

| Example | Nature of clay | ppm levels of Al in eluent liquid during 1h period with 1.0M KOH |
|---------|----------------|------------------------------------------------------------------|
| 1 | silanised alumina pillared interlayered clay (Si:Al=2.8:1) | 78 |
| A | alumina pillared interlayered clay | 1680 |

TABLE 6

| Example | Nature of clay employed | Titre (cm$^3$,0.1MNaOH) to reach pH 7.0 |
|---------|-------------------------|------------------------------------------|
| A | alumina pillared interlayered clay | 0.4 |
| 1 | silanised alumina pillared interlayered clay | 0.9 |

TABLE 7

| Example | Catalyst | Si:Al ratio of catalyst | % wt ethyl benzene in products |
|---------|----------|-------------------------|--------------------------------|
| 7 | Ex. A | 1.3:1 | 1 |
| 8 | Ex. 1 | 2.8:1 | 13 |
| 9 | Ex. 3 | 5.0:1 | 4 |
| 10 | Ex. 4 | 7.0:1 | 2 |

17

0150898

18

### TABLE 8

| Example | Calcination temperature (°C) | wt % ethyl benzene in products |
|---------|------------------------------|--------------------------------|
| 11      | 300                          | 5                              |
| 12      | 360                          | 8                              |
| 13      | 400                          | 13                             |
| 14      | 430                          | 5                              |

### TABLE 9

| Example | Comparison Test 5 | 17 |
|---------|-------------------|-----|
| Nature of catalyst | Alumina pillared interlayered clay steamed at 670°C | Silanised pillared interlayered clay steamed at 670°C |
| Microactivity (%) | 86.4 | 68.2 |
| Gasoline selectivity (wt %) | 53 | 65 |
| Coke yield (wt %) | 18.9 | 8.8 |

### TABLE 10

| Example | Nature of clay catalyst | Wt. ratio of primary: secondary glycol ether in product stream |
|---------|-------------------------|----------------------------------------------------------------|
| 18 | Silanised alumina pillared interlayered clay of Ex. 1 | 1.5 |
| 19 | $Mn^{2+}$ exchanged silanised alumina pillared interlayered clay. | 1.1 |
| 20 | $H^+$ exchanged silanised alumina pillared interlayered clay. | 2.0 |
| Comp. Test 6 | Alumina pillared interlayered clay. | 1.3 |

18

Claims:

1. A silanised pillared interlayered clay comprising a pillared interlayered clay chemically modified by incorporation therein of a silicon-containing residue.

2. A silanised pillared interlayered clay wherein the interlayered clay form which the pillared interlayered clay is derived is a smectite-type clay.

3. A silanised pillared interlayered clay according to either claim 1 or claim 2 wherein the pillared interlayered clay is an alumina pillared interlayered clay.

4. A silanised pillared interlayered clay according to either claim 1 or claim 2 wherein the pillared interlayered clay is a boria pillared interlayered clay.

5. A process for the production of a silanised pillared interlayered clay as claimed in claims 1 to 4 which process comprises hydrolysing a hydrolysable silicon compound in the presence of a pillared interlayered clay.

6. A process according to claim 5 which comprises the steps of:

    (A)    forming the precursor of a pillared interlayered clay,

    (B)    recovering the precursor of the pillared interlayered clay and optionally washing the recovered precursor,

    (C)    calcining the precursor recovered in step (B) to form the pillared interlayered clay, and

    (D)    in the presence of the pillared interlayered clay formed in step (C) hydrolysing a hydrolysable silicon compound.

7. A process according to either claim 5 or claim 6 wherein hydrolysis of the hydrolysable silicon compound is achieved by contacting the compound in the liquid phase with a hydrolysing agent in the presence of the pillared interlayered clay under conditions which do not impair the layered structure of the clay.

19

8.  A process according to claim 7 wherein the hydrolysing agent is water, aqeuous alcohol, dilute aqueous alkali or dilute aqueous alkali.

9.  A process according to either claim 5 or claim 6 wherein hydrolysis of the hydrolysable silicon compound is effected by contacting the pillared interlayered clay with the hydrolysable silicon compound in the vapour phase under conditions which do not impair the layered structure of the clay.

10.  A process according to any one of claims 5 to 9 wherein the hydrolysable silicon compound is a tetraalkoxysilane.

11.  A process according to any one of claims 5 to 10 wherein the silanised pillared interlayered clay is calcined.

12.  A silanised pillared interlayered clay as claimed in any one of claims 1 to 4 when used as a catalyst in the conversion of synthesis gas to hydrocarbons and/or alcohols.

13.  A silanised pillared interlayered clay as claimed in any one of claims 1 to 4 when used as a catalyst in reactions catalysed by protons.

14.  A silanised pillared interlayered clay as claimed in any one of claims 1 to 4 when used as a catalyst in hydrocarbon conversion reactions.